# EUROPEAN PATENT APPLICATION

(11) **EP 4 732 900 A1**
(43) Date of publication of application: **29.04.2026**
(21) Application number: 24826181.0
(22) Date of filing: 11.06.2024
(51) Int. Cl.: A61N 7/02, A61N 7/00

(54) **ULTRASONIC WAVE GENERATING DEVICE HAVING ROTATING ULTRASONIC TRANSDUCER AND ULTRASONIC TREATMENT HANDPIECE COMPRISING SAME**

(30) Priority: 23.06.2023 KR 20230080949
(71) Applicant: CLASSYS INC., Seoul 06220 (KR)
(72) Inventor: PARK, Si Hyung, Seoul 08329 (KR); KIM, Do Yeon, Gunpo-si Gyeonggi-do 15833 (KR)
(74) Representative: Witte, Weller & Partner Patentanwälte mbB
(86) International application number: PCT/KR2024/007959
(87) International publication number: WO 2024/262860

(57) **Abstract**

An ultrasonic wave generating device having a rotating ultrasonic transducer according to an embodiment of the present invention comprises: a cartridge housing portion having a window portion provided on the lower surface thereof such that same contact skin, ultrasonic waves pass through same; an ultrasonic transducer portion positioned in the cartridge housing portion so as to generate ultrasonic waves; a rotatable mounting portion on which the ultrasonic transducer portion is rotatably mounted through axial coupling of a transducer rotating shaft, the rotatable mounting portion being rotated with a preconfigured radius in the cartridge housing portion, thereby moving the ultrasonic transducer portion in a circular shape; and a rotating motor portion for rotating the rotatable mounting portion. The ultrasonic transducer can be moved with a predetermined radius while being rotated such that an ultrasonic focus can be rotated while being moved along a plane inside skin, thereby obtaining various treatment benefits, and twisting of wires for supplying electric power to the ultrasonic transducer can be prevented by rotating the moved ultrasonic transducer with a predetermined radius.

## Description

### Technical Field

The present disclosure relates to an ultrasonic wave generating device having a rotating ultrasonic transducer and an ultrasonic treatment handpiece including the ultrasonic wave generating device. More particularly, the present disclosure relates to an ultrasonic wave generating device having a rotating ultrasonic transducer and an ultrasonic treatment handpiece including the ultrasonic wave generating device capable of moving a focus of ultrasonic waves on a plane within the skin by rotating the ultrasonic transducer and moving the ultrasonic transducer with a preconfigured radius.

### Background Art

Recently, as dietary life has become westernized, with a rapid rise in obesity, obesity has become one of the major causes damaging health and beauty nationally. Accordingly, various diet programs and ultrasonic wave devices for treatment of obesity have been developed and are widely used.

An obesity treatment technique of a High Intensity Focused Ultrasound (HIFU) was originally used for the purpose of anticancer therapy by destroying cancer cells by selectively and noninvasively targeting tumors of internal organs which were coagulated at high temperature. Later, Solta Medical of the USA developed a device called Liposonix in which a HIFU technique is applied and which was the first apparatus used for treatment of human abdominal obesity.

A process of fat-shattering by using the HIFU technique is to cause a tissue temperature to be risen to 65°C to 100°C at a moment when a focused ultrasound is focused on a specific point within a fat layer, thereby destroying the tissue.

An HIFU device induces coagulation necrosis of fat to occur noninvasively by focusing HIFU energy on a selected part without causing any harm on a skin surface, which is different from, for example, laser and high frequency RF equipment used in the dermatology field. The fat necrosed as such is naturally removed by the body's mechanism for repairing damaged areas.

As a known ultrasound obesity treatment device, Korean Patent No. 10-1365946 (Published on Feb 24, 2014), having a title of "HIGH INTENSITY FOCUSED ULTRASOUND GENERATING DEVICE FOR THE DEDUCTION OF FAT TISSUE" has been proposed.

In the "HIGH INTENSITY FOCUSED ULTRASOUND GENERATING DEVICE FOR THE DEDUCTION OF FAT TISSUE", a transducer is moved to a desired position in an X axis direction and a Y axis direction and then is driven by a pivot operation with respect to a shaft, and ultrasonic waves are permeated inside skin.

However, In the "HIGH INTENSITY FOCUSED ULTRASOUND GENERATING DEVICE FOR THE DEDUCTION OF FAT TISSUE", ultrasonic waves are supplied to a curved surface (circular arc) due to the characteristics of the pivot operation when the ultrasonic waves are supplied by the pivot operation, and energy supplied to the skin is reduced and a focus depth is changed when the ultrasonic waves are moved to a periphery, so that there is a problem that a treatment cannot be performed uniformly.

In order to solve this problem, the present applicant has been proposed Korean Patent No. 10-1649899 having a title of "ULTRASONIC APPARATUS FOR TREATMENT". In Korean Patent No. 10-1649899, there has been proposed a structure in which a focus rotational movement unit capable of moving a focus of ultrasonic waves generated from an ultrasound generation unit to be in a circular shape on the same plane is included, and the focus of the ultrasonic waves is formed in the circular shape having a constant radius at a uniform depth in skin and energy is uniformly and evenly applied within the radius, so that a treatment performance is increased.

However, in Korean Patent No. 10-1649899 having the title of "ULTRASONIC APPARATUS FOR TREATMENT", in a structure in which a plurality of protruding members that protrudes at different heights is in contact with an upper surface of the ultrasound generation unit is provided, there is a problem that a limitation in reducing the size of the apparatus and in stably moving the focus of the ultrasonic waves generated from an ultrasonic transducer unit to be in a circular shape on the same plane.

In addition, in Korean Patent No. 10-1649899 having the title of "ULTRASONIC APPARATUS FOR TREATMENT", since the ultrasound generation unit is rotated while the ultrasound generation unit is in an inclined state, ultrasonic waves are generated obliquely and a focus of the ultrasonic waves is formed in an oblique direction on the skin. Therefore, it is difficult for the intensity of the ultrasonic waves, i.e., the output of the ultrasonic waves, to be uniformly delivered to the skin, and it is also difficult to increase a radius of a circle formed on the plane.

In addition, in Korean Patent No. 10-1649899 having the title of "ULTRASONIC APPARATUS FOR TREATMENT", since a structure in which the plurality of protruding members that protrudes at different heights is in contact with an upper surface of the ultrasonic generation unit is provided and the ultrasound generation unit is rotated while the ultrasound generation unit is in the inclined state, the structure becomes complicated. Consequently, manufacturing costs increase significantly, and a torsional moment is generated, resulting in a large load required to rotate the ultrasound generation unit.

Furthermore, in Korean Patent No. 10-1365946 (Published on Feb 24, 2014) having the title of "HIGH INTENSITY FOCUSED ULTRASOUND GENERATING DEVICE FOR THE DEDUCTION OF FAT TISSUE" and in Korean Patent No. 10-1649899 having the title of "ULTRASONIC APPARATUS FOR TREATMENT", since only one ultrasonic transducer is provided, a depth at which a focus of ultrasonic waves reaches is limited to a single depth. As a result, effects of ultrasonic treatment or skin care become limited, and it is difficult to achieve ultrasonic treatment or skin care effects suitable for the condition of a patient's skin.

### Disclosure

### Technical Problem

An objective of the present disclosure is to provide an ultrasonic wave generating device having a rotating ultrasonic transducer and an ultrasonic treatment handpiece including the ultrasonic wave generating device capable of moving and rotating a focus of ultrasonic waves on a plane within the skin by rotating the ultrasonic transducer and moving the ultrasonic transducer with a preconfigured radius.

Another objective of the present disclosure is to provide an ultrasonic wave generating device having a rotating ultrasonic transducer and an ultrasonic treatment handpiece including the ultrasonic wave generating device capable of preventing twisting of a wire by rotating the ultrasonic transducer that is moved with a predetermined radius, the wire being configured to supply power to the ultrasonic transducer.

In addition, still another objective of the present disclosure is to provide an ultrasonic wave generating device having a rotating ultrasonic transducer and an ultrasonic treatment handpiece including the ultrasonic wave generating device capable of moving a focus of ultrasonic waves circularly at a uniform depth in the skin so that the ultrasonic waves can uniformly and evenly penetrate into the skin.

### Technical Solution

In order to achieve the objectives described above, according to an aspect of the present disclosure, there is provided an ultrasonic wave generating device having a rotating ultrasonic transducer, the ultrasonic wave generating device including: a cartridge housing portion having a lower portion provided with a window portion which is brought into contact with the skin and through which ultrasonic waves pass; an ultrasonic transducer portion positioned in the cartridge housing portion and configured to generate ultrasonic waves; a rotatable mounting portion on which the ultrasonic transducer portion is mounted such that the ultrasonic transducer portion is capable of being rotated through axial coupling of a transducer rotating shaft, the rotatable mounting portion being configured to be rotated with a preconfigured radius in the cartridge housing portion, thereby moving the ultrasonic transducer portion circularly; and a rotating motor portion configured to rotate a rotating shaft portion connected to the rotatable mounting portion, wherein the ultrasonic transducer portion revolves about the rotating shaft portion, and rotates about the transducer rotating shaft while being revolved.

According to an aspect of the present disclosure, the ultrasonic wave generating device may further include a transducer rotating portion configured to rotate the ultrasonic transducer portion about the transducer rotating shaft.

In the present disclosure, the transducer rotating portion may include a wire portion for power supply, the wire portion being electrically connected to the ultrasonic transducer portion, the wire portion being configured to supply electric power to the ultrasonic transducer portion, and the wire portion being configured to control an operation of the ultrasonic transducer portion.

In the present disclosure, the ultrasonic transducer portion may be connected to the wire portion for power supply, and may be configured to be rotated about the transducer rotating shaft while being in a state in which a point of the ultrasonic transducer portion where the wire portion for power supply is connected is positioned toward a fixed orientation.

In the present disclosure, the wire portion for power supply may have a stretchable wire structure or a stretchable power cable structure.

In the present disclosure, the wire portion for power supply may be a spring coil wire portion which is wound in a spiral shape and which is elastically stretchable.

In the present disclosure, the transducer rotating portion may further include a coil retaining rod portion which stands upright and which is positioned through an inner portion of the spring coil wire portion.

In the present disclosure, the rotatable mounting portion may include a mounting arm member having an upper end portion connected to the rotating shaft portion and having a lower end portion connected to the ultrasonic transducer portion via the transducer rotating shaft, and the mounting arm member may be disposed obliquely.

In the present disclosure, the transducer rotating shaft may be positioned at an eccentric position spaced apart from a center of the ultrasonic transducer portion, so that a focus of ultrasonic waves may be moved circularly with a radius that is a distance spaced apart from the transducer rotating shaft by a rotation of the ultrasonic transducer portion.

In the present disclosure, the ultrasonic transducer portion may include a plurality of ultrasonic transducers and a transducer mounting body portion on which the plurality of ultrasonic transducers is mounted and which is coupled to the rotatable mounting portion such that the transducer mounting body portion is capable of being rotated through the transducer rotating shaft.

In the present disclosure, at least one of the plurality of ultrasonic transducers may have a different position at which a focus of ultrasonic waves reaches.

In the present disclosure, the transducer rotating portion may include a transducer rotating motor configured to rotate the ultrasonic transducer portion.

### Advantageous Effects

In the present disclosure, a focus of ultrasonic waves is capable of being rotated while being moved on a plane within the skin by rotating and moving the ultrasonic transducer with the preconfigured radius, so that various treatment effects may be achieved.

In addition, in the present disclosure, since twisting of the wire that supplies power to the ultrasonic transducer may be prevented by rotating the ultrasonic transducer that is moved with the preconfigured radius, there are effects that electric power may be stably supplied to the ultrasonic transducer portion through the wire and the operational stability may be achieved.

In addition, in the present disclosure, an energy is uniformly and evenly applied to a treatment area by moving a focus of ultrasonic waves in a plane at a uniform depth in the skin, and the focus of the ultrasonic waves is formed in a circular shape having a constant radius at the uniform depth in the skin, so that there is an effect that a treatment performance is increased by uniformly and evenly applying the energy within the radius.

### Description of Drawings

FIG. 1 is a perspective view illustrating an embodiment of an ultrasonic treatment handpiece according to the present disclosure.
FIG. 2 is an exploded perspective view illustrating an embodiment of the ultrasonic treatment handpiece according to the present disclosure.
FIG. 3 is a cross-sectional view illustrating an embodiment of an ultrasonic wave generating device having a rotating ultrasonic transducer according to the present disclosure.
FIG. 4 is a bottom view illustrating an embodiment of the ultrasonic wave generating device having the rotating ultrasonic transducer according to the present disclosure.
FIG. 5 is a cross-sectional view illustrating another embodiment of the ultrasonic wave generating device having the rotating ultrasonic transducer according to the present disclosure.
FIG. 6 is a bottom view illustrating another embodiment of the ultrasonic wave generating device having the rotating ultrasonic transducer according to the present disclosure.
FIG. 7 is a cross-sectional view illustrating still another embodiment of the ultrasonic wave generating device having the rotating ultrasonic transducer according to the present disclosure.
FIG. 8 is a bottom view illustrating still another embodiment of the ultrasonic wave generating device having the rotating ultrasonic transducer according to the present disclosure.

### *Description of Reference Numerals*

100: Body housing portion 101: Cartridge locking portion
110: Handle portion 111: Handle connection portion
112: Handle body 200: Cartridge housing portion
201: Upper housing portion 201a: Cartridge board portion
202: Housing sealing cap portion 203: Window portion
210: Protruding tube portion for medium supply 220: Protruding tube portion for medium discharge
300: Ultrasonic transducer portion 310: First ultrasonic transducer
320: Second ultrasonic transducer 330: Transducer mounting body portion
400: Rotating motor portion 410: Shaft adapter portion
420: Rotating shaft portion 421: Connection shaft portion
500: Rotatable mounting portion 510: Mounting arm member
600: Transducer rotating portion 600a: Transducer rotating shaft
610: Wire portion for power supply 611: Spring coil wire portion
620: Coil retaining rod portion 630: Transducer rotating motor
700: Shaft supporting plate portion 800: Supporting rod portion

### Mode for Invention

Hereinbelow, the present disclosure will be described in more detail.

An exemplary embodiment of the present disclosure will be described in detail with reference to the accompanying drawings. Prior to the detailed description of the present disclosure, it should be noted that the terms and words used in the specification and the claims should not be construed as being limited to ordinary meanings or dictionary definitions. Therefore, the description proposed herein is just an exemplary embodiment for the purpose of illustrations only, not intended to limit the scope of the present disclosure, so it should be understood that other equivalents and modifications could be made thereto without departing from the scope of the present disclosure at the time at which the present application is filed.

Hereinbelow, the present disclosure will be described in more detail.

An exemplary embodiment of the present disclosure will be described in detail with reference to the accompanying drawings. Prior to the detailed description of the present disclosure, it should be noted that the terms and words used in the specification and the claims should not be construed as being limited to ordinary meanings or dictionary definitions. Therefore, the description proposed herein is just an exemplary embodiment for the purpose of illustrations and not intended to limit the scope of the present disclosure, so it should be understood that other equivalents and modifications could be made thereto without departing from the scope of the present disclosure at the time at which the present application is filed.

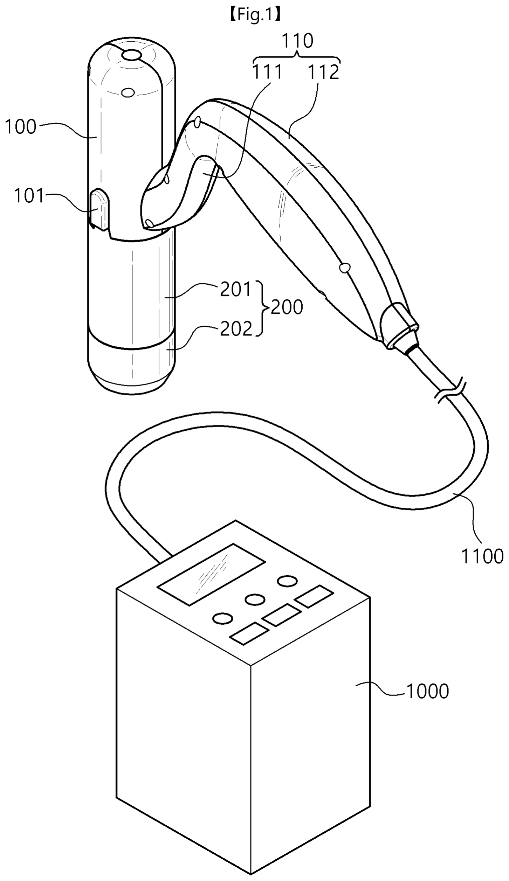

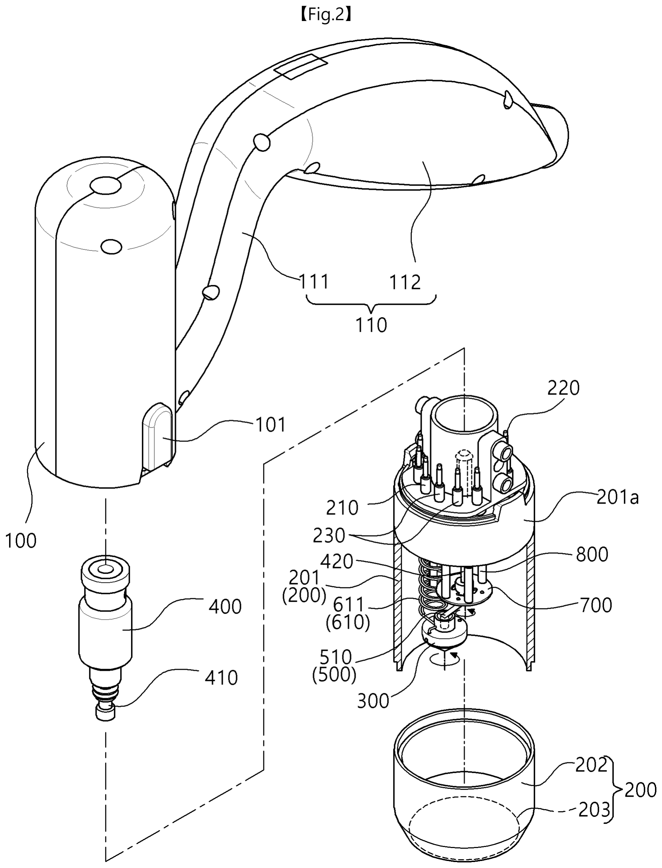

FIG. 1 is a perspective view illustrating an embodiment of an ultrasonic treatment handpiece according to the present disclosure, and FIG. 2 is an exploded perspective view illustrating an embodiment of the ultrasonic treatment handpiece according to the present disclosure.

Referring to FIG. 1 to FIG. 2, the ultrasonic treatment handpiece according to the present disclosure includes a cartridge housing portion 200 in which an ultrasonic transducer portion 300 is positioned, and includes a body housing portion 100 to which an ultrasonic wave generating device configured to allow ultrasonic waves to pass through a window portion 203 positioned at a lower portion of the cartridge housing portion 200 so that the ultrasonic waves irradiate the skin is coupled such that the ultrasonic wave generating device is capable of being attached to and detached from the body housing portion 100.

The ultrasonic treatment handpiece according to the present disclosure is connected to a control body 1000 via a cable body 1100 for a handpiece, the cable body 1100 including a power cable and both a medium supply line and a medium discharge line that are configured to circulate a medium in the cartridge housing portion 200. Furthermore, the cable body 1100 for the handpiece and the control body 1000 may be implemented in various forms of known examples of known ultrasonic treatment devices, so that detailed descriptions thereof will be omitted.

The ultrasonic wave generating device, i.e., the cartridge housing portion 200, is coupled to the body housing portion 100 such that the ultrasonic wave generating device is capable of being attached to and detached from the body housing portion 100, and is capable of being replaced after the ultrasonic wave generating device is used for a predetermined number of times.

In addition, the ultrasonic wave generating device includes an embodiment having a rotating ultrasonic transducer according to the present disclosure, the embodiment being described in detail below.

A cartridge locking portion 101 on which the cartridge housing portion 200 is capable of being hung and fixed is positioned at the body housing portion 100. Furthermore, the cartridge locking portion 101 is elastically supported by a spring, and a wedge-shaped catching portion is caught in a catching groove that is positioned inside the cartridge housing portion 200, so that a coupled state of the cartridge housing portion 200 is fixed. Furthermore, when the cartridge locking portion 101 is pressed, the catching portion is separated from the catching groove, and the cartridge housing portion 200 is capable of being detached from the cartridge locking portion 101.

In addition to the cartridge locking portion 101, known locking structures capable of separating the cartridge housing portion 200 may be applied to the cartridge housing portion 200, and the cartridge housing portion 200 may be maintained in a state of being coupled to the body housing portion 100 or may be detached from the body housing portion 100. Therefore, a detailed description of the cartridge housing portion 200 will be omitted.

A handle portion 110 that an operator can hold is positioned on one side of the body housing portion 100.

The handle portion 110 includes a handle connection portion 111 bent toward an upper side of the body housing portion 100, and includes a handle body 112 bent downward from the handle connection portion 111.

The operator may hold the handle body 112 which is bent from the handle connection portion 111 and which integrally extends downward, and may perform a treatment by easily bringing the window portion 203 of the cartridge housing portion 200 into close contact with to skin.

The handle portion 110 is designed such that a portion of the handle portion 110 having a shape which is bent upward and then which extends and is bent downward, i.e., the handle body 112, is held. Therefore, a load applied to the operator during the treatment may be minimized, and the treatment may be performed with the window portion 203 of the cartridge housing portion 200 being capable of being easily in close contact with skin.

Meanwhile, the ultrasonic wave generating device having the rotating ultrasonic transducer includes the cartridge housing portion 200 having a lower surface provided with the window portion 203 which is in contact with the skin and through which ultrasonic waves pass, and includes the ultrasonic transducer portion 300 positioned in the cartridge housing portion 200 and configured to generate ultrasonic waves.

The window portion 203 is manufactured from a transparent material or a translucent material through which ultrasonic waves penetrate. Furthermore, since the window portion 203 is manufactured from known materials through which ultrasonic waves penetrate, a detailed description of the window portion 203 will be omitted.

The cartridge housing portion 200 includes an upper housing portion 201 coupled to the body housing portion 100 such that the upper housing portion 201 is capable of being attached to and detached from the body housing portion 100, and includes a housing sealing cap portion 202 coupled to a lower portion of the upper housing portion 201 such that the housing sealing cap portion 202 is capable of being attached to and detached from the lower portion of the upper housing portion 201.

The window portion 203 having a size capable of performing an ultrasonic treatment by being in contact with the skin of a patient is formed on a lower surface of the housing sealing cap portion 202.

The cartridge housing portion 200 has a structure in which an inner portion of the cartridge housing portion 200 is sealed, and the inner portion of the cartridge housing portion 200 is filled with a medium for transmitting ultrasonic waves.

The medium for transmitting ultrasonic waves may be an insulating liquid as an example, and the insulating liquid may be variously modified and implemented as any known electrically non-conductive insulating liquid, so that a detailed description thereof will be omitted.

The medium for transmitting ultrasonic waves may not only serve to transmit ultrasonic waves but may also serve to cool the skin of the patient through the window portion 203 that is in contact with the skin.

A cartridge board portion 201a sealing the inner portion of the cartridge housing portion 200 is positioned on an upper portion of the cartridge housing portion 200, and a protruding tube portion 210 for medium supply and a protruding tube portion 220 for medium discharge that are for circulating the medium for transmitting ultrasonic waves protrude on an upper surface of the cartridge board portion 201a.

As an example, a rotating motor portion 400 includes a rotating shaft portion 420 mounted on an upper surface of a rotatable mounting portion 500, and the rotating shaft portion 420 protrudes upward on the upper surface of the rotatable mounting portion 500 and is mounted at a rotational center of the rotatable mounting portion 500 such that the rotating shaft portion 420 is capable of being attached to and detached from the rotating motor portion 400.

In the cartridge housing portion 200, the rotatable mounting portion 500 on which the ultrasonic transducer portion 300 is mounted such that the ultrasonic transducer portion 300 is capable of being rotated is positioned such that the rotatable mounting portion 500 is capable of being moved circularly with a preconfigured radius by being moved with respect to the rotating shaft portion 420.

The rotating shaft portion 420 is positioned in the cartridge housing portion 200 such that the rotating shaft portion 420 stands upright at a central portion of the cartridge housing portion 200, has a lower end portion connected to the rotatable mounting portion 500 on which the ultrasonic transducer portion 300 is mounted such that the ultrasonic transducer portion 300 is capable of being rotated, and has a part of an upper end portion which protrudes to an upper surface of the cartridge board portion 201a and which is connected to the rotating motor portion 400.

The rotating motor portion 400 is connected to the rotating shaft portion 420 and is configured to transmit a rotational force to the rotating shaft portion 420, so that the rotatable mounting portion 500 on which the ultrasonic transducer portion 300 is mounted such that the ultrasonic transducer portion 300 is capable of being rotated is rotated with respect to the rotating shaft portion 420.

The ultrasonic transducer portion 300 is mounted on the rotational center of the rotatable mounting portion 500. That is, the ultrasonic transducer portion 300 is mounted on the rotatable mounting portion 500 at an eccentric position spaced apart from a rotational center to which the rotating shaft portion 420 is connected.

The rotatable mounting portion 500 may include a mounting arm member 510 connected to the rotational center to which the rotating shaft portion 420 is connected. That is, the rotatable mounting portion 500 may include the mounting arm member 510 connected to a lower end portion of the rotating shaft portion 420 and configured to be rotated integrally with the rotating shaft portion 420 so as to be moved circularly.

The ultrasonic transducer portion 300 is mounted on an end portion of the mounting arm member 510 such that the ultrasonic transducer portion 300 is capable of being rotated, and is mounted on a transducer rotating shaft 600a such that the ultrasonic transducer portion 300 is capable of being rotated, the transducer rotating shaft 600a protruding vertically from a lower end portion of the mounting arm member 510.

A bearing is provided between the transducer rotating shaft 600a and the ultrasonic transducer portion 300, so that the ultrasonic transducer portion 300 is capable of being smoothly rotated about the transducer rotating shaft 600a.

In summary, the ultrasonic transducer portion 300 is positioned such that the ultrasonic transducer portion 300 is spaced apart from the rotational center of the rotatable mounting portion 500, and is mounted on the rotatable mounting portion 500 such that the ultrasonic transducer portion 300 is capable of being rotated by the transducer rotating shaft 600a, so that the ultrasonic transducer portion 300 is configured to be rotated about the transducer rotating shaft 600a while the ultrasonic transducer portion 300 travels in a circular path at the preconfigured radius from the rotational center of the rotatable mounting portion 500.

That is, the ultrasonic transducer portion 300 is configured to revolve about the rotating shaft portion 420, and is configured to rotate about the transducer rotating shaft 600a while being revolved.

In addition, an embodiment of the ultrasonic wave generating device having the rotating ultrasonic transducer according to the present disclosure further includes a transducer rotating portion 600 configured to rotate the ultrasonic transducer portion 300 that is mounted on the rotatable mounting portion 500 such that the ultrasonic transducer portion 300 is capable of being rotated.

The transducer rotating portion 600 includes a wire portion 610 for power supply, the wire portion 610 being electrically connected to the ultrasonic transducer portion 300, the wire portion 610 being configured to supply an electrical power to the ultrasonic transducer portion 300, and the wire portion 610 being configured to control an operation of the ultrasonic transducer portion 300.

The wire portion 610 for power supply is connected to the ultrasonic transducer portion 300, and is configured such that the ultrasonic transducer portion 300 is rotated about the transducer rotating shaft 600a when the rotatable mounting portion 500 is rotated and the ultrasonic transducer portion 300 is moved circularly with the predetermined radius.

The wire portion 610 for power supply has a slack length such that a state in which the wire portion 610 for power supply is connected to the ultrasonic transducer portion 300 is maintained when the ultrasonic transducer portion 300 is moved circularly with the preconfigured radius.

In addition, since the wire portion 610 for power supply has a flexible wire structure or a flexible power cable structure, the ultrasonic transducer portion 300 is rotated by tension of a wire or a cable, i.e., the ultrasonic transducer portion 300 is rotated about the transducer rotating shaft 600a by the tension of the wire or the cable.

In summary, the wire portion 610 for power supply has a structure of a wire or a power cable in which a length is elastically adjustable, and the tension of the wire or the cable causes the ultrasonic transducer portion 300 to rotate, i.e., to rotate about the transducer rotation shaft 600a.

As an example, the wire portion 610 for power supply is a spring coil wire portion 611 which is wound in a spiral shape and which is capable of being elastically stretched.

Alternatively, although not illustrated, the wire portion 610 for power supply may have a structure in which the wire or the power cable is wound on a winding roll elastically supported, so that the wire or the power cable is wound on the winding roll again by elasticity after the wire or the power cable is unwound from the winding roll.

The wire portion 610 for power supply serves to hold the ultrasonic transducer portion 300 at a connection point. Therefore, when the ultrasonic transducer portion 300 is moved circularly with the preconfigured radius by a rotation of the rotatable mounting portion 500, the ultrasonic transducer portion 300 is capable of being rotated about the transducer rotating shaft 600a.

Meanwhile, the ultrasonic transducer portion 300 is positioned such that a lower surface of the ultrasonic transducer portion 300 from which generated ultrasonic waves are emitted is positioned in a direction perpendicular to the window portion 203, i.e., positioned parallel to the window portion 203. Therefore, the ultrasonic transducer portion 300 is configured to emit ultrasonic waves in a direction perpendicular to the window portion 203.

The ultrasonic transducer portion 300 is positioned such that the ultrasonic transducer portion 300 generates ultrasonic waves in a direction perpendicular to the window portion 203 that is in contact with the skin, so that the ultrasonic transducer portion 300 is advantageous in manufacturing and output measurement. Furthermore, the ultrasonic transducer portion 300 may generate ultrasonic waves while being moved circularly, and may be driven with a relatively small load since no torsional moment is generated.

The rotating motor portion 400 is positioned in the body housing portion 100. Furthermore, when the cartridge housing portion 200 is coupled to the lower portion of the body housing portion 100, a portion of the rotating shaft portion 420, which protrudes toward the upper portion of the cartridge housing 200 is connected to the rotating motor portion 400. That is, a portion of the rotating shaft portion 420, which protrudes toward the upper surface of the cartridge board portion 201a is connected to the rotating motor portion 400.

As an example, the rotating motor portion 400 is positioned in the body housing portion 100 and is connected to the rotating shaft portion 420 when the cartridge housing portion 200 is coupled to the lower portion of the body housing portion 100, so that replacement cost when the cartridge housing portion 200 is replaced according to the service life of the ultrasonic transducer portion 300 may be reduced.

Although not illustrated, the rotating motor portion 400 may have a structure in which the rotating motor portion 400 is mounted in the cartridge housing portion 200 and the rotating motor portion 400 is replaced together with the cartridge housing portion 200 when the cartridge housing portion 200 is replaced according to the service life of the ultrasonic transducer portion 300.

The rotating shaft portion 420 protrudes toward the upper surface of the cartridge board portion 201a, and is positioned such that the rotating shaft portion 420 is capable of being rotated.

The rotating shaft portion 420 penetrates the cartridge board portion 201a and is positioned such that the rotating shaft portion 420 is capable of being rotated, and may be implemented by using a known sealing structure that seals a rotating shaft, so that a more detailed description thereof will be omitted.

The rotating shaft portion 420 includes a connection shaft portion 421 that protrudes toward the upper portion of the cartridge board portion 201a, i.e., the upper portion of the cartridge housing portion 200. Furthermore, a shaft of the rotating motor portion 400 is provided with a shaft adapter portion 410 into which the connection shaft portion 421 is inserted such that the connection shaft portion 421 is connected to the shaft adapter portion 410.

A shaft insertion portion that is open downward is positioned inside the shaft adapter portion 410 such that the connection shaft portion 421 is inserted into an inner portion of the shaft insertion portion. As an example, the connection shaft portion 421 is a shaft having a polygonal cross-sectional area, and the shaft insertion portion is an insertion groove portion having a polygonal shape that corresponds to the connection shaft portion 421.

A supply tube connection portion (not illustrated) and a discharge tube connection portion (not illustrated) that connect the protruding tube portion 210 for medium supply and the protruding tube portion 220 for medium discharge to a medium circulating portion (not illustrated) positioned in the control body 1000 when the body housing portion 100 and the cartridge housing portion 200 are coupled to each other are positioned in the body housing portion 100, the control body 1000 being configured to control an operation of the ultrasonic treatment handpiece.

The control body 1000 may be implemented in various forms in a known ultrasonic treatment device which includes a control portion configured to control an operation of an ultrasonic treatment handpiece and which includes a medium circulating portion configured to circulate a medium for transmitting ultrasonic waves, so that a more detailed description thereof will be omitted.

Although the medium circulating portion is not illustrated, the medium circulating portion may be implemented in various forms by using a known cooling water circulating structure including a medium storage tank, a medium supply line portion connecting the medium storage tank to a supply tube connection portion, a medium discharge line portion connecting the medium storage tank to a discharge tube connection portion, a valve positioned at the medium supply line portion, a medium cooling portion positioned at the medium storage tank, and so on, so that a more detailed description thereof will be omitted.

The supply tube connection portion (not illustrated) is provided with a first protruding tube insertion portion into which the protruding tube portion 210 for medium supply is inserted, and the discharge tube connection portion (not illustrated) is provided with a second protruding tube insertion portion into which the protruding tube portion 220 for medium discharge is inserted.

As an example, the protruding tube portion 210 for medium supply is inserted into the first protruding tube insertion portion, and a flow path thereof is open, so that the protruding tube portion 210 for medium supply is connected to the medium supply line portion. Furthermore, as an example, the protruding tube portion 220 for medium discharge is inserted into the second protruding tube insertion portion, and a flow path thereof is open, so that the protruding tube portion 220 for medium discharge is connected to the medium discharge line portion.

Both the protruding tube portion 210 for medium supply and the supply tube connection portion (not illustrated) and both the protruding tube portion 220 for medium discharge and the discharge tube connection portion (not illustrated) may be implemented in various forms by applying a known tube connection structure including a valve which connects two tubes to each other and which is open when the two tubes are connected to each other.

When the cartridge housing portion 200 is coupled to the body housing portion 100, the connection shaft portion 421 of the rotating shaft portion 420 is inserted into the shaft insertion portion of the shaft adapter portion 410, so that the rotating shaft portion 420 and the shaft of the rotating motor portion 400 are connected to each other. Furthermore, the protruding tube portion 210 for medium supply is connected to the medium circulating portion of the control body 1000 by being inserted into the first protruding tube insertion portion of the supply tube connection portion, and the protruding tube portion 220 for medium discharge is connected to the medium circulating portion of the control body 1000 by being inserted into the second protruding tube insertion portion of the discharge tube connection portion.

In addition, a pair of first main power supply terminals 230 for supplying power to the ultrasonic transducer portion 300 is provided on the upper surface of the cartridge housing portion 200, i.e., on the upper surface of the cartridge board portion 201a. Furthermore, although not illustrated, a pair of second main power supply terminals to which the pair of first main power supply terminals 230 is connected and which is connected to the control body 1000 via the cable body 1100 for the handpiece is positioned in the body housing portion 100.

As an example, the first main power supply terminal 230 is a protrusion-type terminal that protrudes on the upper surface of the cartridge housing portion 200, i.e., on the upper surface of the cartridge board portion 201a. Furthermore, the second main power supply terminal is an insertion-type terminal into which the first main power supply terminal 230 is capable of being inserted and connected.

When the cartridge housing portion 200 is coupled to the body housing portion 100, the pair of first main power supply terminals 230 is inserted into the pair of second main power supply terminals such that the pair of first main power supply terminals 230 and the pair of second main power supply terminals are connected to each other, and the ultrasonic transducer portion 300 is connected to the control body 1000 via the cable body 1100 for the handpiece, so that the ultrasonic transducer portion 300 may receive power from the control body 1000 and the operation of the ultrasonic transducer portion 300 may be controlled.

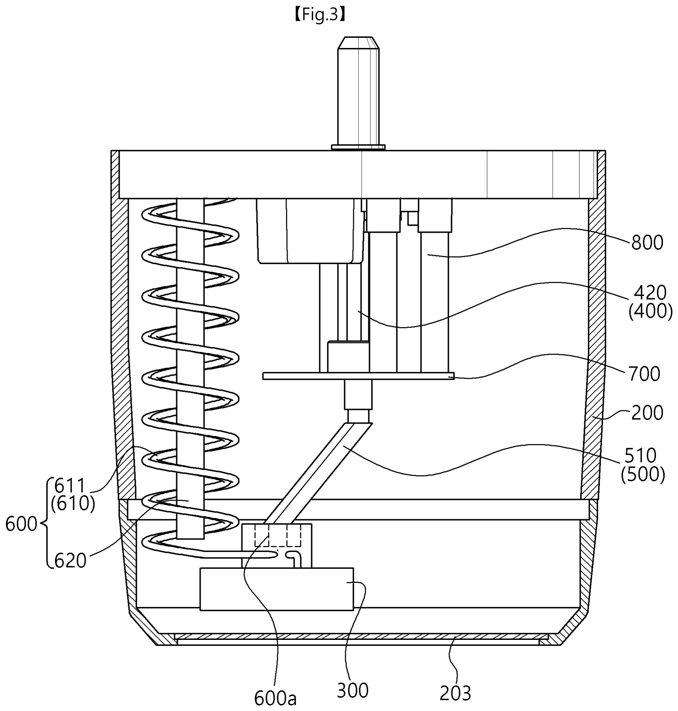

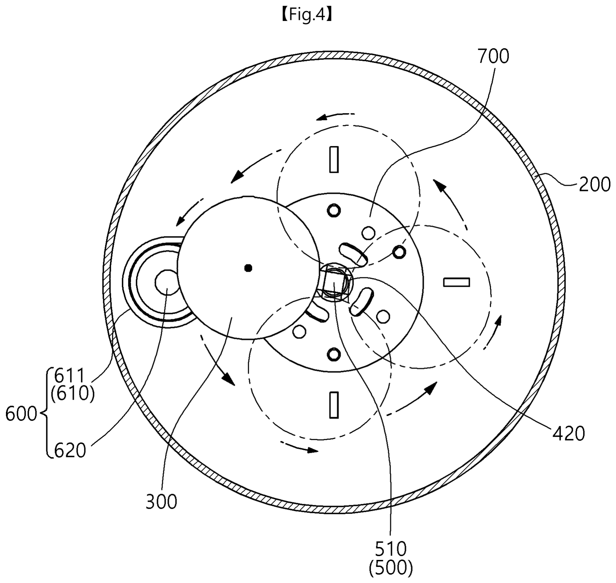

Meanwhile, FIG. 3 is a cross-sectional view illustrating an embodiment of an ultrasonic wave generating device having a rotating ultrasonic transducer according to the present disclosure, and FIG. 4 is a bottom view illustrating an embodiment of the ultrasonic wave generating device having the rotating ultrasonic transducer according to the present disclosure.

Referring to FIG. 3 and FIG. 4, an embodiment of the ultrasonic wave generating device having the rotating ultrasonic transducer according to the present disclosure includes a shaft supporting plate portion 700 through which the rotating shaft portion 420 passes and which supports a rotation of the rotating shaft portion 420, and includes a plurality of supporting rod portions 800 having first side ends fixed to the upper surface of the cartridge housing portion 200 and having lower end portions fixed to the shaft supporting plate portion 700 so as to support a position of the shaft supporting plate portion 700.

The rotating shaft portion 420 penetrates the shaft supporting plate portion 700 and is positioned such that the rotating shaft portion 420 is capable of being rotated, so that the rotating shaft portion 420 is configured to be stably rotated at an inner center of the cartridge housing portion 200.

The shaft supporting plate portion 700 is fixed to the inner portion of the cartridge housing portion 200 by the plurality of supporting rod portions 800, and the plurality of supporting rod portions 800 stably supports the position of the shaft supporting plate portion 700.

A portion of the rotating shaft portion 420 protrudes from a lower portion of the shaft supporting plate portion 700, and is connected to the rotatable mounting portion 500.

The mounting arm member 510, which is the rotatable mounting portion 500, has an upper end portion connected to the rotating shaft portion 420, and the ultrasonic transducer portion 300 is positioned at a lower end portion of the mounting arm member 510 such that the ultrasonic transducer portion 300 is capable of being rotated.

As an example, the mounting arm member 510 is formed in a rod shape having a cylindrical cross-sectional area or a polygonal cross-sectional area, and is disposed obliquely so that the wire portion 610 for power supply is not to be caught and twisted on the mounting arm member 510 when the mounting arm member 510 is moved circularly with the preconfigured radius.

The wire portion 610 for power supply is the spring coil wire portion 611 which is wound in the spiral shape and which is capable of being elastically stretched, and the transducer rotating portion 600 further includes a coil retaining rod portion 620 which is positioned upright so as to pass through an inner portion of the spring coil wire portion 611.

When the spring coil wire portion 611 is pulled by a movement of the ultrasonic transducer portion 300 and a length of the spring coil wire portion 611 is increased, the coil retaining rod portion 620 guides the spring coil wire portion 611 to be sequentially unwound from a lower end portion of the spring coil wire portion 611 connected to the ultrasonic transducer portion 300. Furthermore, when the spring coil wire portion 611, which has been extended by the movement of the ultrasonic transducer portion 300, is reduced again by an elastic force, the coil retaining rod portion 620 guides the spring coil wire portion 611 to be rewound in a reverse order.

The coil retaining rod portion 620 supports a position of the spring coil wire portion 611 that is unwound or wound while the ultrasonic transducer portion 300 is moved circularly on a plane, so that a coil shape of the spring coil wire portion 611 is maintained even when the spring coil wire portion 611 is repeatedly unwound or wound.

The ultrasonic transducer portion 300 is connected to the wire portion 610 for power supply so as to supply power to the ultrasonic transducer portion 300 and to control the operation of the ultrasonic transducer portion 300, and is configured to emit ultrasonic waves while being moved circularly on the plane with the preconfigured radius by the rotation of the rotating shaft portion 420, so that a focus of ultrasonic waves emitted by the ultrasonic transducer portion 300 is moved circularly on a plane.

As the ultrasonic transducer portion 300 is moved circularly on the plane, the ultrasonic transducer portion 300 pulls or unwinds the spring coil wire portion 611, and the ultrasonic transducer portion 300 is rotated about the transducer rotating shaft 600a by an elastic force of the spring coil wire portion 611, the elastic force being generated by such pulling or unwinding. Therefore, a point at which the spring coil wire portion 611 is connected to the ultrasonic transducer portion 300 is always positioned toward a fixed orientation, thereby preventing the spring coil wire portion 611 from being twisted.

The ultrasonic transducer portion 300 is moved circularly on the plane with a radius that is a distance spaced apart from the rotational center of the rotatable mounting portion 500, so that a focus of ultrasonic waves generated by each ultrasonic transducer portion 300 is moved such that the focus forms a circle on the same plane.

The ultrasonic transducer portion 300 is rotated about the rotating shaft portion 420 in a state in which an ultrasonic wave generating surface of the ultrasonic transducer portion 300 from which ultrasonic waves are emitted is positioned parallel to the window portion in a direction perpendicular to the window portion 203. That is, the ultrasonic transducer portion 300 is rotated about the rotating shaft portion 420 in a state in which the ultrasonic wave generating surface is positioned horizontally. Therefore, a focus of ultrasonic waves generated from the ultrasonic transducer portion 300 is moved circularly on the same plane.

That is, in the ultrasonic wave generating device having the rotating ultrasonic transducer according to the present disclosure, a focus of each ultrasonic wave generated in the ultrasonic transducer portion 300 is positioned at a uniform depth in the skin and is moved on the same plane with the preconfigured radius, so that a movement path is formed in a circular shape on the plane and energy is uniformly and evenly applied within the radius, thereby further increasing treatment performance.

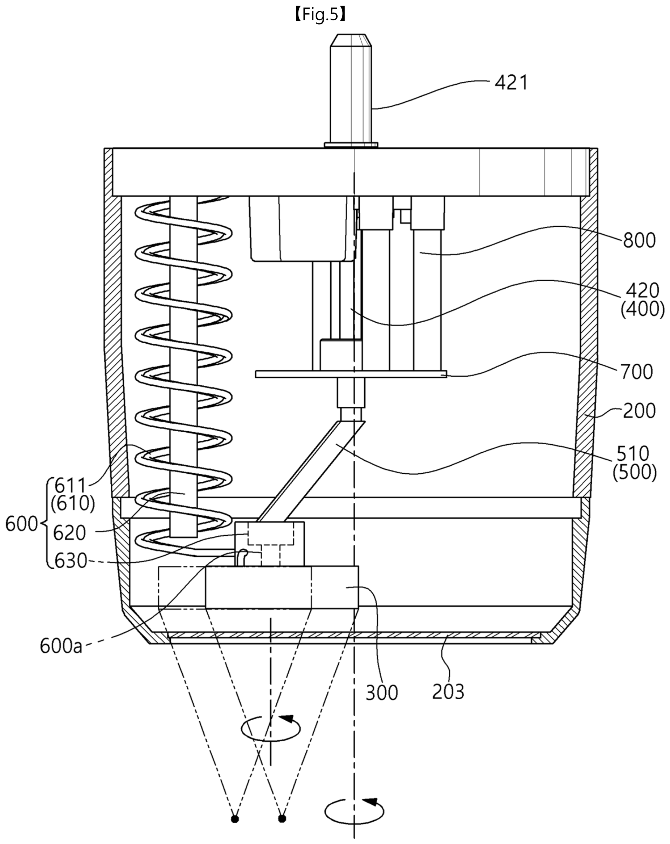

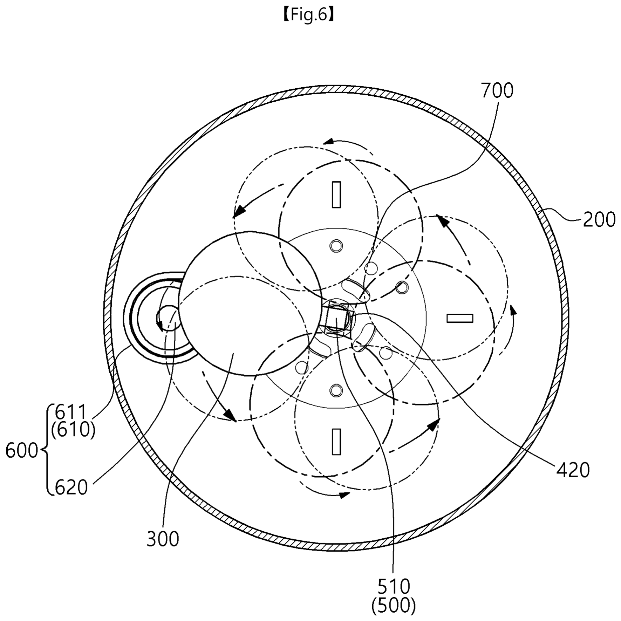

Meanwhile, FIG. 5 is a cross-sectional view illustrating another embodiment of the ultrasonic wave generating device having the rotating ultrasonic transducer according to the present disclosure, and FIG. 6 is a bottom view illustrating another embodiment of the ultrasonic wave generating device having the rotating ultrasonic transducer according to the present disclosure.

Referring to FIG. 5 and FIG. 6, the transducer rotating shaft 600a, which is the rotating shaft of the ultrasonic transducer portion 300, is positioned at the center of the ultrasonic transducer portion 300, i.e., positioned at an eccentric position centered on a focus of ultrasonic waves generated in the ultrasonic transducer portion 300. Therefore, the focus of the ultrasonic waves may be moved circularly by the rotation of the ultrasonic transducer portion 300, and the focus forming the circular path may be moved on the same plane with the preconfigured radius.

The ultrasonic transducer portion 300 is moved circularly on a plane with a radius that is a distance spaced apart from the rotational center of the rotatable mounting portion 500 by the rotation of the rotatable mounting portion 500. Here, the ultrasonic transducer portion 300 is rotated by the transducer rotating portion 600.

The ultrasonic transducer portion 300 is moved circularly with the preconfigured radius by the rotation of the rotatable mounting portion 500, and is rotated about the transducer rotating shaft 600a by the elastic force of the spring coil wire portion 611. Here, since the transducer rotating shaft 600a is positioned eccentrically with respect to the focus of the ultrasonic waves, the focus is moved circularly about the transducer rotating shaft 600a with a radius corresponding to an eccentric distance.

In addition, the transducer rotating portion 600 may further include a transducer rotating motor 630 positioned at an end portion of the rotatable mounting portion 500 and configured to rotate the ultrasonic transducer portion 300.

The transducer rotating motor 630 may be operated by receiving an electric power by the wire portion 610 for power supply, or may be operated by receiving electric power through a brush structure.

The transducer rotating motor 630 is configured to rotate the ultrasonic transducer portion 300, so that even the wire portion 610 for power supply, which does not have an elastic structure, may efficiently be prevented from being twisted due to the circular movement of the ultrasonic transducer portion 300, and the ultrasonic transducer portion 300 that is rotatable in an eccentric position with respect to the focus of the ultrasonic waves may be rotated with a second radius.

That is, when the ultrasonic transducer portion 300 is moved circularly with the predetermined radius by the rotation of the rotatable mounting portion 500, the focus of the ultrasonic waves is moved circularly with a first radius by the rotation of the rotatable mounting portion 500, and the focus is also moved circularly with the second radius by the rotation of the ultrasonic transducer portion 300, the second radius being eccentric with respect to the transducer rotating shaft 600a.

Therefore, in another embodiment of the ultrasonic wave generating device having the rotating ultrasonic transducer according to the present disclosure, the focus of the ultrasonic waves may be moved circularly with a large first radius on the plane, and may be moved circularly with a small second radius on the plane, so that a variety of ultrasonic treatment may be achieved and suitable ultrasonic treatment may be performed according to a patient.

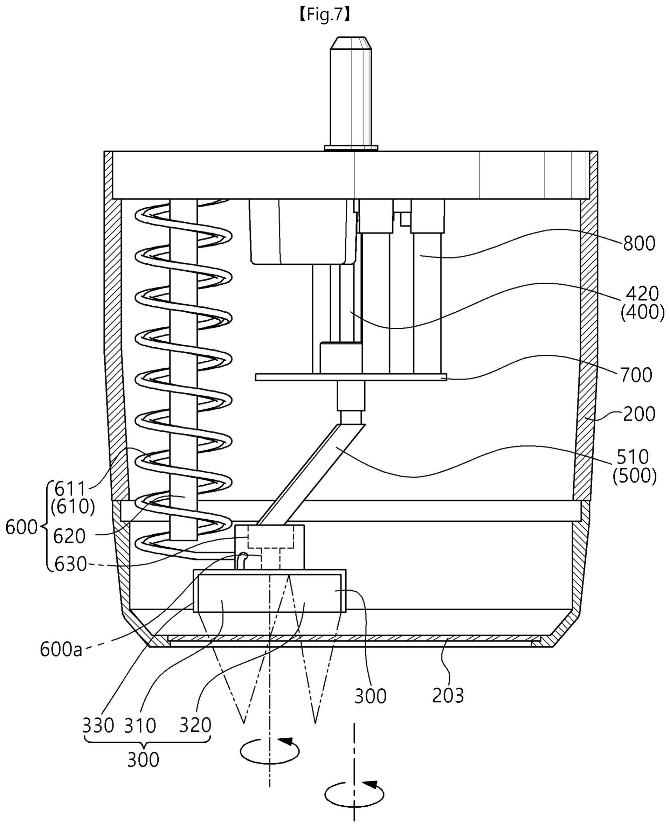

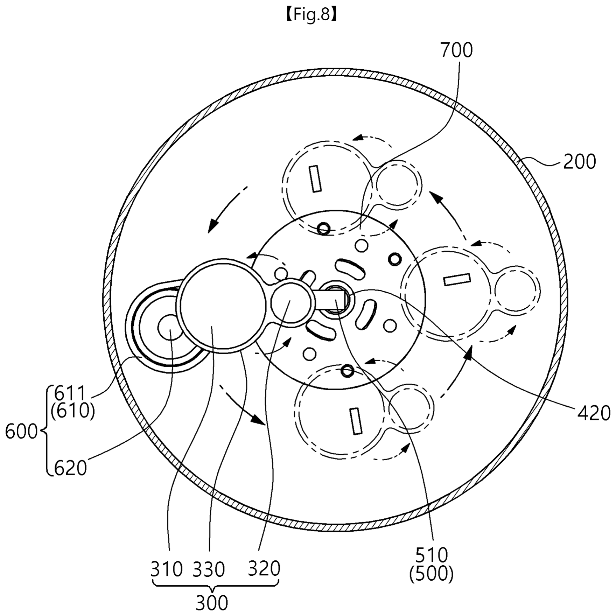

In addition, FIG. 7 is a cross-sectional view illustrating still another embodiment of the ultrasonic wave generating device having the rotating ultrasonic transducer according to the present disclosure, and FIG. 8 is a bottom view illustrating still another embodiment of the ultrasonic wave generating device having the rotating ultrasonic transducer according to the present disclosure.

The ultrasonic transducer portion 300 may include a plurality of ultrasonic transducers 310 and 320 (a first ultrasonic transducer 310 and a second ultrasonic transducer 320), and may include a transducer mounting body portion 330 on which the plurality of ultrasonic transducers 310 and 320 is mounted and which is coupled to the rotatable mounting portion 500 such that the transducer mounting body portion 330 is capable of being rotated by the transducer rotating shaft 600a. Therefore, in the ultrasonic transducer portion 300, as the transducer mounting body portion 330 is rotated about the transducer rotating shaft 600a while the plurality of ultrasonic transducers 310 and 320 is moved circularly by the rotation of the rotatable mounting portion 500 with a radius that is a distance spaced apart from the rotational center of the rotatable mounting portion 500, each of the ultrasonic transducers 310 and 320 may be moved circularly with a radius that is a distance spaced apart from the transducer rotating shaft 600a.

Each of the plurality of ultrasonic transducers 310 and 320 may have a structure in which a focus of ultrasonic waves is positioned at the same position, i.e., at the same depth. Furthermore, at least one of the plurality of ultrasonic transducers 310 and 320 may have a structure in which a focus of ultrasonic waves is positioned at a different position, i.e., at a different depth. Therefore, since there is a difference in a depth at which a focus of the ultrasonic waves reaches, a treatment effect on the skin at various depths may be achieved.

In addition, the transducer rotating portion 600 may further include the transducer rotating motor 630 positioned at the end portion of the rotatable mounting portion 500 and configured to rotate the ultrasonic transducer portion 300. That is, the transducer rotating motor 630 is configured to rotate the transducer mounting body portion 330.

The transducer rotating motor 630 may be operated by receiving an electric power by the wire portion 610 for power supply, or may be operated by receiving electric power through a brush structure.

The transducer rotating motor 630 is configured to rotate the transducer mounting body portion 300, so that even the wire portion 610 for power supply, which does not have an elastic structure, may efficiently be prevented from being twisted due to the circular movement of the ultrasonic transducer portion 300. Furthermore, as the transducer rotating motor 630 rotates the transducer mounting body portion 330 about the transducer rotating shaft 600a, the plurality of ultrasonic transducers 310 and 320 having the center spaced apart from the transducer rotating shaft 600a is moved circularly with a radius that is a distance spaced apart from the transducer rotating shaft 600a.

The plurality of ultrasonic transducers 310 and 320 is mounted at respective eccentric positions on the rotatable mounting portion 500 that is rotated by the rotating motor portion 400, so that each focus generated from the ultrasonic transducer portion 300 is moved circularly on the same plane.

In addition, since each of the plurality of ultrasonic transducers 310 and 320 has a structure in which the center of each of the ultrasonic transducers 310 and 320 is positioned eccentrically with respect to the transducer rotating shaft 600a, the focus of the ultrasonic waves emitted from each of the ultrasonic transducers 310 and 320 is rotated about the transducer rotating shaft 600a and is moved circularly.

That is, each of the ultrasonic transducers 310 and 320 is moved such that each of the ultrasonic transducers 310 and 320 is moved circularly with a radius that is a distance spaced apart from the rotational center of the rotatable mounting portion 500, and is also moved circularly with a radius that is a distance spaced apart from the transducer rotating shaft 600a.

In addition, the focus of each of the ultrasonic transducers 310 and 320 is moved such that the focus of each of the ultrasonic transducers 310 and 320 is moved circularly with a radius that is a distance spaced apart from the rotational center of the rotatable mounting portion 500, and is also moved circularly with a radius that is a distance spaced apart from the transducer rotating shaft 600a.

More specifically, the ultrasonic transducer portion 300 includes the first ultrasonic transducer 310 and the second ultrasonic transducer 320, and the transducer mounting body portion 330 is moved circularly with a radius corresponding to a horizontal projection length of the rotatable mounting portion 500. Furthermore, the first ultrasonic transducer 310 is moved circularly along a circular shape having a radius corresponding to the horizontal projection length of the rotatable mounting portion 500, and is also moved circularly with a radius corresponding to a first distance that is a distance spaced apart from the transducer rotating shaft 600a. Furthermore, the second ultrasonic transducer 320 is moved circularly along a circular shape having a radius corresponding to the horizontal projection length of the rotatable mounting portion 500, and is also moved circularly with a radius corresponding to a second distance that is a distance spaced apart from the transducer rotating shaft 600a.

Therefore, a focus of first ultrasonic waves emitted from the first ultrasonic transducer 310 is moved circularly with a radius that is a distance spaced apart from the rotational center of the rotatable mounting portion 500, and is also moved circularly with a radius that is the first distance spaced apart from the transducer rotating shaft 600a.

Therefore, a focus of second ultrasonic waves emitted from the second ultrasonic transducer 320 is moved circularly with a radius that is a distance spaced apart from the rotational center of the rotatable mounting portion 500, and is also moved circularly with a radius that is the second distance spaced apart from the transducer rotating shaft 600a.

Therefore, in still another embodiment of the ultrasonic wave generating device having the rotating ultrasonic transducer according to the present disclosure, since each focus of ultrasonic waves emitted from the plurality of ultrasonic transducers 310 and 320 is capable of being moved circularly on the plane with the large first radius and is also capable of being moved circularly on the plane with the small second radius, a variety of ultrasonic treatment may be achieved, appropriate ultrasonic treatment according to a patient may be performed, and the variety of ultrasonic treatment may further be achieved when the depth of each focus of ultrasonic waves is different from each other, so that patient satisfaction and the treatment effect may be significantly increased.

In the present disclosure, a focus of ultrasonic waves is capable of being rotated while being moved on a plane within the skin by rotating and moving the ultrasonic transducer with the preconfigured radius, so that various treatment effects may be achieved.

In addition, in the present disclosure, since twisting of the wire that supplies power to the ultrasonic transducer may be prevented by rotating the ultrasonic transducer that is moved with the preconfigured radius, electric power may be stably supplied to the ultrasonic transducer portion 300 through the wire, and the operational stability may be achieved.

In addition, in the present disclosure, energy is uniformly and evenly applied to a treatment region by moving a focus of ultrasonic waves on a plane at a uniform depth in the skin, and the focus of the ultrasonic waves is moved circularly with the preconfigured radius at the uniform depth in the skin, so that the energy is uniformly and evenly applied within the radius, thereby being capable of increasing the treatment performance.

It is to be understood that the present disclosure is not limited to the above described embodiments but may be variously modified and embodied within the scope of the present disclosure without departing from the gist of the present disclosure.

## Claims

1. An ultrasonic wave generating device having a rotating ultrasonic transducer, the ultrasonic wave generating device comprising:
a cartridge housing portion having a lower portion provided with a window portion which is brought into contact with a skin and through which ultrasonic waves pass;
an ultrasonic transducer portion positioned in the cartridge housing portion and configured to generate ultrasonic waves;
a rotatable mounting portion on which the ultrasonic transducer portion is mounted such that the ultrasonic transducer portion is capable of being rotated through axial coupling of a transducer rotating shaft, the rotatable mounting portion being configured to be rotated with a preconfigured radius in the cartridge housing portion, thereby moving the ultrasonic transducer portion circularly; and
a rotating motor portion configured to rotate a rotating shaft portion connected to the rotatable mounting portion,
wherein the ultrasonic transducer portion revolves about the rotating shaft portion, and rotates about the transducer rotating shaft while being revolved.

2. The ultrasonic wave generating device of claim 1, further comprising:
a transducer rotating portion configured to rotate the ultrasonic transducer portion about the transducer rotating shaft.

3. The ultrasonic wave generating device of claim 2, wherein the transducer rotating portion comprises a wire portion for power supply, the wire portion being electrically connected to the ultrasonic transducer portion, the wire portion being configured to supply electric power to the ultrasonic transducer portion, and the wire portion being configured to control an operation of the ultrasonic transducer portion.

4. The ultrasonic wave generating device of claim 3, wherein the ultrasonic transducer portion is connected to the wire portion for power supply, and is configured to be rotated about the transducer rotating shaft while being in a state in which a point of the ultrasonic transducer portion where the wire portion for power supply is connected is positioned toward a fixed orientation.

5. The ultrasonic wave generating device of claim 3, wherein the wire portion for power supply has a stretchable wire structure or a stretchable power cable structure.

6. The ultrasonic wave generating device of claim 3, wherein the wire portion for power supply is a spring coil wire portion which is wound in a spiral shape and which is elastically stretchable.

7. The ultrasonic wave generating device of claim 6, wherein the transducer rotating portion further comprises a coil retaining rod portion which stands upright and which is positioned through an inner portion of the spring coil wire portion.

8. The ultrasonic wave generating device of claim 3, wherein the rotatable mounting portion comprises a mounting arm member having an upper end portion connected to the rotating shaft portion and having a lower end portion connected to the ultrasonic transducer portion via the transducer rotating shaft, and the mounting arm member is disposed obliquely.

9. The ultrasonic wave generating device of claim 2, wherein the transducer rotating shaft is positioned at an eccentric position spaced apart from a center of the ultrasonic transducer portion, so that a focus of ultrasonic waves is moved circularly with a radius that is a distance spaced apart from the transducer rotating shaft by a rotation of the ultrasonic transducer portion.

10. The ultrasonic wave generating device of claim 2, wherein the ultrasonic transducer portion comprises:
a plurality of ultrasonic transducers; and
a transducer mounting body portion on which the plurality of ultrasonic transducers is mounted and which is coupled to the rotatable mounting portion such that the transducer mounting body portion is capable of being rotated through the transducer rotating shaft.

11. The ultrasonic wave generating device of claim 10, wherein at least one of the plurality of ultrasonic transducers has a different position at which a focus of ultrasonic waves reaches.

12. The ultrasonic wave generating device of claim 2, wherein the transducer rotating portion comprises a transducer rotating motor configured to rotate the ultrasonic transducer portion.
